# EUROPÄISCHE PATENTSCHRIFT

(11) **EP 1 866 634 B1**
(45) Veröffentlichungstag und Bekanntmachung des Hinweises auf die Patenterteilung: **29.06.2011**
(21) Anmeldenummer: 06725452.4
(22) Anmeldetag: 30.03.2006
(51) Int. Cl.: G01N 27/403

(54) **ABWASSERANALYSE-SENSORKARTUSCHE**
WASTEWATER ANALYSIS SENSOR CARTRIDGE
CARTOUCHE DE DETECTION A ANALYSE DES EAUX USEES

(30) Priorität: 08.04.2005 EP 05102787
(43) Veröffentlichungstag der Anmeldung: 19.12.2007
(73) Patentinhaber: Hach Lange GmbH, 14163 Berlin (DE)
(72) Erfinder: UTHEMANN, Rolf, 51373 Leverkussen (DE); GOLITZ, Andreas, 47445 Moers (DE); WOODWARD, John, R., Windsor, CO 80550 (US)
(74) Vertreter: Eberlein, Jasper
(86) Internationale Anmeldenummer: PCT/EP2006/061204
(87) Internationale Veröffentlichungsnummer: WO 2006/106071

(56) Entgegenhaltungen:
- DE-A1- 4 234 021
- DE-A1- 4 406 908
- US-A- 4 495 050

## Beschreibung

Die Erfindung bezieht sich auf eine Abwasseranalyse-Sensorkartusche mit Sensorelementen mit ionenselektiven Membranen und für den einmaligen Gebrauch.

Sensoren für die Analyse von Abwasser in Kläranlagen u.ä. sind schon aufgrund der unwirtlichen Messumgebung, nämlich Abwasser, relativ anfällig für Störungen. Ferner ist für elektro-chemische Sensorelemente bzw. für Referenzelemente jeweils ein Vorrat an Elektrolyt in einem Elektrolyttank vorzusehen. Bei Ausfall des Sensor-elementes oder des Peferenzelemelites, oder aber bei einem leeren Elektrolyttank, muss das betreffende Sensor- bzw. Referenzelement ausgetauscht bzw. der betreffende Elektrolyttank mit Elektrolyt aufgefüllt werden. Die Feststellung, welche Sensorelement bzw. Referenzelement ausgetauscht werden muss, ist bei Fehlfunktionen häufig nicht einfach zu treffen. Das Austauschen einzelner Sensorelemente bzw. das Auffüllen des Elcktrolyttanks ist umständlich, zeitaufwändig und kostenintensiv.

Aus DE 295 08 870 U1 ist eine Sensoranordnung bekannt, die mehrere pH-Sensorelemente aufweist. Das Gehäuse und die Elektrolyttanks werden von mehreren koaxial ineinander angeordneten Glas-Rohren gebildet. Das Gehäuse einschließlich der Elektrolyttanks aus Glas ist in der Herstellung sehr aufwändig, bei der Handhabung bruchanfällig und für Sensorelemente mit ionenselektiven Membranen, die zur Messung von anderen Parametern als dem pH-Wert erforderlich sind, ungeeignet.

Aus DE 83 19 463 U ist eine Sensorkartusche bekannt, bei der die verschiedenen Tanks und Gehäuse durch verschiedene separate Bauteile aus Glas und/oder Kunststoff gebildet werden. Die Sensorkartusche muss aus den genannten Bauteilen zusammengebaut werden. Der Zusammenbau ist aufwändig und fehierträchtig. Die flüssigkeitsdichte Abdichtung der Bauteile aus verschiedenen Materialien ist schwierig und aufwendig.

Eine pH- Sensoranordnung mit einem einstückigen Kunststoff gehäuse wird in US 4495050 beschrieben. Aufgabe der Erfindung ist es demgegenüber, die Herstellung und die Eigenschaften einer Abwasser-Sensorkartusche mit ionenselektiven Membranen zu vereinfachen bzw. zu verbessern.

Diese Aufgabe wird erfindungsgemäß gelöst mit den Merkmalen des Patentanspruches 1.

Gemäß der Erfindung ist eine Abwasseranalyse-Sensorkartusche für den einmaligen Gebrauch vorgesehen. Die Sensorkartusche weist hierzu ein einstückiges flüssigkeitsdichtes Gehäuse auf, das aus Kunststoff gefertigt ist. Das Gehäuse enthält mindestens zwei elektro-chemische Sensorelemente und mindestens ein Referenzelement. Ferner enthält das einstückige Gehäuse zwei Sensorelement-Elektrolyttanks und einen Referenzelement-Elektrolyttank, in denen sich das für das Sensorelement bzw. das Referenzelement benötigte Elektrolyt befindet. Sobald eines der Elemente defekt ist, oder einer der Elektrolyttanks leer ist, wird die gesamte Sensorkartusche ausgetauscht gegen eine neue Sensorkartusche, die vollständig gefüllte Elektrolyttanks aufweist und neue Sensorelemente und ein neues Referenzelement enthält. Eine Austauschbarkeit des Sensorelementes oder des Referenzelementes oder eine Auffüllbarkeit eines Elektrolyttanks ist nicht vorgesehen.

Die umständliche Suche nach dem defekten Element bzw. der umständliche Austausch einzelner Elemente entfällt. Auch das umständliche Auffüllen eines Elektrolyttanks entfällt. Die Lebensdauer der Sensorelemente und des Referenzelementes kann u.U. auf das Volumen des Elektrolyttanks abgestimmt werden. Die Sensorelementen und das Referenzelement müssen nicht länger halten, als der Vorrat in den Elektrolyttanks ausreicht. Je nach Elementart steckt hierin ein weiteres Einsparpotenzial.

Das einstückige Gehäuse bildet die Elektrolyttanks. Das Gehäuse weist innenseitig eine komplexe Struktur auf, die mehrere geschlossene oder verschließbare Räume zur Verfügung stellt. In dem Gehäuse lassen sich auf diese Weise praktisch ohne Mehrkosten mehrere Elektrolyttanks bilden. Derartige Strukturen lassen sich problemlos und preiswert beispielsweise im Kunststoff-Spritzguss herstellen.

Die Sensorkartusche kann in großen Stückzahlen hergestellt werden, so dass die Herstellungskosten relativ gering sind. Da zum Austausch der Sensorelemente und des Referenzelementes und zum Auffüllen der Elektrolyttanks nur noch wenige Handgriffe erforderlich sind, sind die Wartungs- und Instandhaltungskosten für eine Abwasseranalyse-Anlage, in der die Sensorkartusche zum Einsatz kommt, niedriger als für herkömmliche Abwasseranalyse-Anlagen.

Jedes elektro-chemische Sensorelement weist eine ionenselektive Kunststoff-Membran auf, die den Sensor-Elektrolyttank abschließt und die jeweils in einer Öffnung der Außenwand und des Kunststoff-Gehäuses angeordnet ist. Durch die Verwendung von Kunststoff, insbesondere von PVC, wird sowohl für das Gehäuse als auch die ionenselektive Sensorelement-Membran ein gleichartiges Material verwendet.

Unter einer ionenselektiven Kunststoff-Membran wird sowohl eine Kunststoff-Membran verstanden, in die ionenaustauschende Substanzen eingelagert sind, als auch sogenannte "Solid state"-Membranen, in die relativ unlösliche anorganische Salze eingelagert sind.

Durch die Verwendung eines gleichartigen Materiales für die Membranen einerseits und das Gehäuse andererseits wird die flüssigkeitsdichte Abdichtung der Grenze der zwischen der Membran und dem Gehäuse vereinfacht und zuverlässiger gemacht. Dies hat u.a. damit zu tun, dass aufgrund der Materialgleichheit oder -ähnlichkeit das Gehäuse und die Membran ähnliche Ausdehnungskoeffizienten aufweisen.

Vorzugsweise sind die ionenselektiven Kunststoff-Membranen stoffschlüssig mit dem Kunststoff-Gehäuse verbunden. Die stoffschlüssige Verbindung kann beispielsweise durch sogenanntes Kaltverschweißen mit einem lösungsmittelhaltigen Kleber erfolgen. Erst die Verwendung von Kunststoff sowohl für das Gehäuse als auch für die ionenselektive Membran ermöglicht eine derartige stoffschlüssige und vollständige flüssigkeitsdichte Verbindung zwischen der ionenselektiven Membran und dem Gehäuse- Alternativ können auch HF-Schweißen oder andere Verfahren zur Herstellung einer stoffschlüssigen Verbindung eingesetzt werden.

Gemäß einer bevorzugten Ausgestaltung bildet die Gehäusewand des Gehäuses jeweils teilweise die Tankwand der Elektrolyttanks. Die Tankwände der Elektrolyttanks und die Gehäusewand des Gehäuses überlappen sich mindestens teilweise. Auf diese Weise kann ein Teil der Wände gemeinsam als Gehäusewand und als Tankwand fungieren. Hierdurch wird im Innenraum des Gehäuses insgesamt mehr Hohlraum gewonnen, der für die Elektrolyttanks genutzt werden kann. Durch die Überlappung der Wände der Elektrolyttanks miteinander und/oder mit der Seitenwand des Gehäuses können die Elektrolyttanks bei unveränderter Gehäusegröße in ihrem Tankvolumen vergrößert werden.

Gemäß einer bevorzugten Ausgestaltung ist das Gehäuse becherförmig ausgestaltet, wobei das bzw. die Sensorelementen und das Stromschlüssel-Element und ggf. der Temperatursensor am Becherboden angeordnet sind. Die Becheröffnung wird durch einen separaten Deckel verschlossen, der mit dem Becher verklebt oder verschweißt oder auf andere Weise flüssigkeitsdicht verbunden ist. Der Becherdeckel weist die elektrischen Kupplungselemente auf.

Vorzugsweise ist in den Öffnungen der Elektrolyttanks jeweils eine fluiddurchlässige Tragstruktur vorgesehen, wobei jede Tragstruktur- eine Membrane des betreffenden Sensorelementes trägt. Die Membrane des Sensorelementes liegt außenseitig auf der Tragstruktur auf und wird durch diese gestützt und getragen.

Das elektro-chemische Sensorelement kann beispielsweise ein Ammonium-Sensorelement und/oder ein Kalium-Sensorelement zur Kompensation der Querempfindlichkeit des Ammonium-Sensorelementes sein.

Das Referenzelement kann als pH-Sonde ausgebildet sein, die zusammen mit dem Referenzelement-Elcktrolyt und einem Stromschlüssel-Element eine Referenzanordnung bildet.

Gemäß einer bevorzugten Ausgestaltung weist das Gehäuse eine elektro-mechanische Kupplungsanordnung auf, mit der es an eine entsprechende elektro-mechanische Kupplungsanordnung einer landseitig befestigten Haltevorrichtung kuppelbar ist. Die Kupplungsanordnung dient zum einen der mechanischen Befestigung der Sensorkartusche an der Haltevorrichtung, beispielsweise einem Halterahmen, der landseitig neben dem Abwasserbecken montiert ist und mit einem entsprechenden Haltearm in das Klärbecken bzw. in das Abwasser hineinragt. Zum anderen dient die Kupplungsanordnung der elektrischen Verbindung des Sensorelementes und des Referenzelementes mit der landseitigen Anlage, in der die elektrischen Signale des Sensorelementes und des Referenzelementes ausgewertet werden.

Gemäß einer bevorzugten Ausgestaltung weist die Kupplungsanordnung ein Dichtelement auf, so dass sie mit der Kupplungsanordnung der landseitig befestigten Haltevorrichtung flüssigkeitsdicht zusammenkuppelbar ist. Der innere Bereich der aneinander gekuppelten Kupplungsanordnungen ist flüssigkeitsdicht isoliert gegenüber der Umgebung, so dass die elektrischen Verbindungen zwischen den beiden Kupplungsanordnungen keiner Feuchtigkeit bzw. Flüssigkeit ausgesetzt sind.

Vorzugsweise trägt das Gehäuse einen Temperatursensor. Auch ein Stromschlüssel-Element für das Referenzelement, beispielsweise in Form einer Saizbrücke, kann von dem Gehäuse getragen werden.

Im Folgenden wird unter Bezugnahme auf die Zeichnungen ein Ausführungsbeispiel der Erfindung näher erläutert.

Es zeigen:
- Figur 1: eine Abwasseranalyse-Anlage mit einer erfindungsgemäßen Abwasseranalyse-Sensorkartusche,
- Figur 2: die Abwasseranalyse-Sensorkartusche der Figur 1 im Querschnitt,
- Figur 3: die Abwasseranafyse-Sensorkartusche der Figur 1 im Längsschnitt,
- Figur 4: die Abwasseranalyse-Sensorkatusche der Figur 1 in perspektivischer Ansicht von unten, und
- Figur 5: die Abwasseranalyse-Sensorkanusche der Figuren 1 bis 4 von der Seite der Kupplungsanordnung aus in perspektivischer Ansicht.

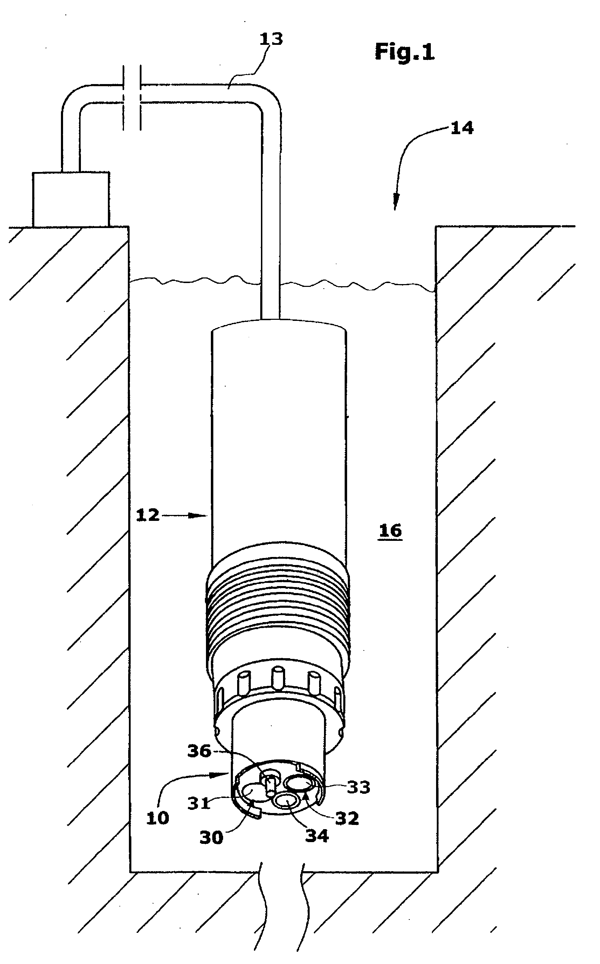

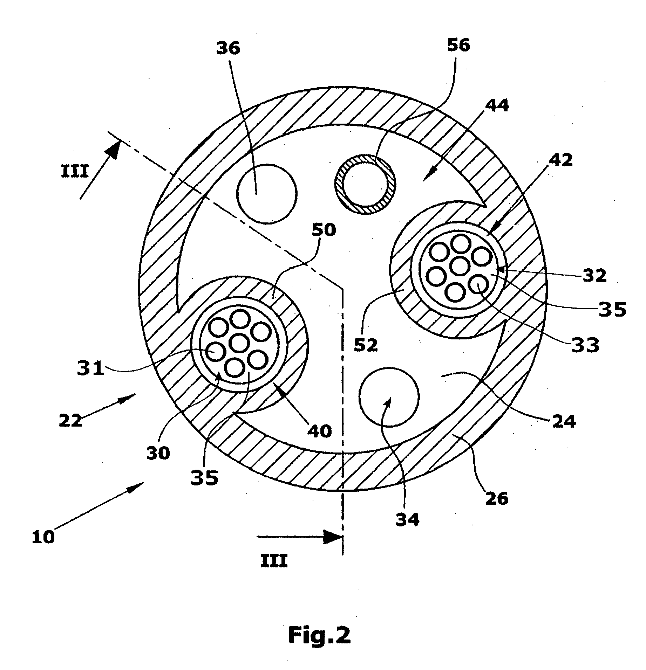

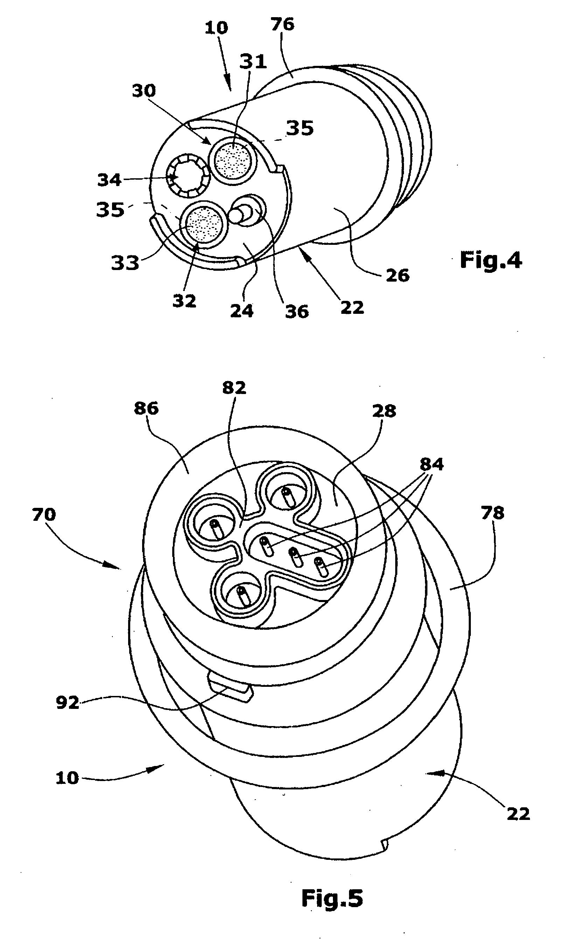

In den Figuren 1 bis 5 ist eine Abwasseranalyse-Sensorkartusche 10 dargestellt, die an einer landseitig befestigten Haltevorrichtung 12 abnehmbar befestigt ist. Die Haltevorrichtung 12 ist zusammen mit der Sensorkartusche 10 in ein Abwasserbecken 14 einer Kläranlage vollständig eingetaucht.

Die Sensorkartusche 10 besteht aus einem einstückigen flüssigkeitsdichten Kunststoff-Gehäuse 20, das wiederum aus einem einstückigen Gehäusebecher 22 mit einer Bodenwand 24, einer Seitenwand 26 sowie einer mit dem Gehäusebecher 22 verklebten oder verschwcißten Deckelwand 28 besteht. Der Gehäusebecher 22 ist annähernd zylindrisch ausgebildet. Die Bodenwand 24 und die Seitenwand 26 bilden eine Außenwand des Gehäuses 20.

In der Bodenwand 24 des Bechers 22 sind zwei elektro-chemische Sensorelemente 30,32, ein Stromschlüssel-Element 34 sowie ein Temperatursensor 36 angeordnet. In das Gehäuse 20 bzw. den Becher 22 ragt ein stabförmiges Referenzelement 56 senkrecht von oben hinein.

Das erste elektro-cherrische Sensorelement 30 dient der clcktro-chemischen Messung von Ammonium, das zweite Sensorelement 32 dient der elektrochemischen Messung von Kalium zur Kompensation der Querempfindlichkeit der Ammonium-Messung. Das Stromschlüssel-Element 34 ist als sog. Salzbrücke ausgebildet, die als Spannungsüberträger dient, und ein flüssiges oder gelartiges Elektrolyt zwischen zwei Diaphragmen aufweist. Der Temperatursensor 36 dient der Ermittlung der Abwasser-Temperatur.

Den beiden Sensorelementen 30,32 und dem Referenzelement 56 ist jeweils ein Elektrolyttanks 40,42,44 zugeordnet, der von Tankwänden 50,52 in dem Gehäuse 20 gebildet wird. An die Gehäuseseitenwand 26 schließen sich die zwei zylindrischen Tankwände 50,52 an, die sich mit der Gehäuseseitenwand 26 teilweise überschneiden, so dass die Gehäuseseitenwand 26 teilweise die Tankwand 50 der beiden nicht weiter ausgekleideten Elektrolyttanks 40,42 bildet. Der verbleibende Innenraum innerhalb des Gehäuses 20 und außerhalb des Elektrolyttanks 40,42 bildet den Elektrolyttank 44 des Referenzelementes 56. Durch die Überschneidungen der Gehäuseseitenwand 26 und der Tankwände 50,52 der Elektrolyttanks 40,42 wird ein größtmögliches Volumen des Referenzelement-Elektrolyttanks 44 ermöglicht.

Jedes elektro-chemische Sensorelement 30,32 weist jeweils eine ionenselektiven Kunststoff-Membran 31,33 auf, die den betreffenden Sensor-Elektrolyttank 40,42 abschließt und die jeweils in einer Öffnung der Außenwand des Gehäuses 20 angeordnet ist. Die ionenselektive Kunststoff-Membran 31,33 wird jeweils durch ein Kunststoff-Plättchen gebildet, in das Ionophore eingelagert sind, die definieren, welche Ionen in die Membran 31,33 eindringen können. Beide Kunststoff-Membranen 31,33 sind durch Kaltverschweißen mit Hilfe eines lösungsmittelhaltigen Klebers stoffschlüssig mit dem Kunststoff-Gehäuse 20 verbunden. Hierdurch wird eine absolut flüssigkeitsdichte Abdichtung sichergestellt. Die ionenselektive Kunststoff-Membran 31,33 kann alternativ jeweils auch als "Solid state"-Membran ausgebildet sein, in die relativ unlösliche anorganische Salze eingelagert, sind.

In der Öffnung, in der die beiden ionenselektiven Membranen 31,33 sitzen, ist ferner jeweils eine fluiddurchlässige Tragstruktur 35 innenseitig angeordnet, die die betreffende ionenselektive Membran 31,33 innenseitig gegen den Flüssigkeitsdruck abstützt. Die Tragstruktur 35 weist zur Herstellung der Fluiddurchlässigkeit relativ kleine Öffnungen auf, und ist beispielsweise als Gitter-Struktur oder durchlöcherte Struktur ausgebildet. Die Tragstruktur- 35 besteht ebenfalls aus Kunststoff.

Das Referenzelement 56 ist als pH-Sonde ausgebildet, die von dem Deckel 28 aus axial Richtung Becher-Bodenwand 24 herabhängt und in das Elektrolyt eingetaucht ist. Das Referenzelement 56, das Elektrolyt in dem Referenzelement-Elektrolyttank 44 und das Stromschlüssel-Element 34 bilden zusammen eine Referenzanordnung, die ein Referenzsignal für die von den beiden Sensorelementen 30,32 gewonnenen Sensorsignale liefert.

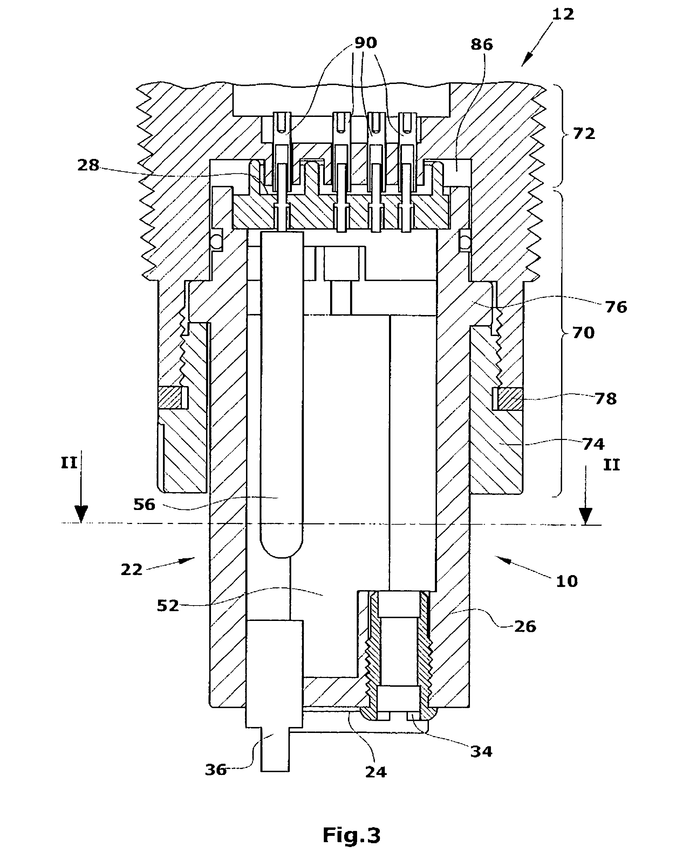

An dem dem Becherboden 24 gegenüberliegenden Längsende des Gehäuses 20 ist eine elektro-mechanische Kupplungsanordnung 70 vorgesehen, die mit einer entsprechenden elektro-mechanischen Kupplungsanordnung 72 der Haltevorrichtung 12 zusammenkuppelbar ist, wie in Figur 3 dargestellt. Die Kupplungsanordnungen 70,72 sind flüssigkeitsdicht miteinander kuppelbar.

Die Kupplungsanordnung 70 der Sensorkartusche 10 weist im Wesentlichen eine Überwurfmutter 74, einen Überwurfmutter-Flansch 76, einen Überwurfmuttern-Dichtungsring 78, einen Radialdichtungsring 80 sowie von Kontaktschutzwänden 82 umgebene Kontaktstifte 84 und einen Abschlussdichtungsring 86 auf.

Die Kontaktstifte 84 sind in entsprechende Kontaktbuchsen 90 der Kupplungsanordnung 72 der Haltevorrichtung 12 eingesteckt. Die Montage bzw. Demontage der Sensorkartusclie 10 an bzw. von der Haltevorrichtung 12 erfolgt durch entsprechendes Festschrauben bzw. Abschrauben der Überwurfmutter 74. Ferner sind als Verdrehsicherungen zwei Sicherungsnasen 92 an dem Gehäuse vorgesehen, die in entsprechende Ausnehmungen der Haltevorrichtung 12 eingreifen.

Die Haltevorrichtung 12 weist ein flüssigkeitsdichtes Gehäuse auf, in dem sich die Messelektronik befindet. Durch die Nähe der Messelektronik zu den Sensorelementen und dem Referenzelement können die relativ schwachen Messsignale zuverlässig und genau ausgewertet werden. Die Haltevorrichtung kann an einem Halterohr 13 steif und ortsfest montiert, kann aber auch an einem Kabel hängend aufgehängt sein.

Bei Fehlfunktion, bei rechnerisch ermittelter kurz bevorstehender und/oder signalisierter Erschöpfung eines der Elektrolyte wird die auszutauschende Sensorkartusche 10 nach Lösen der Überwurfmutter 74 abgezogen und eine neue Sensorkartusche 10 durch Ankoppeln und Festschrauben der Überwurfmutter ausgetauscht. Eine zeitaufwändige Fehlersuche, ein Austausch einzelner Elemente oder ein umständliches manuelles Auffüllen der Elektrolyte entfällt.

## Patentansprüche

1. Abwasseranalyse-Sensorkartusche (10) für den einmaligen Gebrauch, mit
einem einstückigen Gehäuse (20) aus Kunststoff, das mindestens zwei elektro-chemische Sensorelemente (30,32) und mindestens ein Referenzelement (56) aufweist, und
zwei Sensorelement-Elektrolyttanks (40,42) und einen Referenzelement-Elektrolyttank (44), wobei
alle Elektrolyttanks (40, 42, 44) von dem einstückigen Gehäuse (20) gebildet werden, und
jedes elektro-chemische Sensorelement (30,32) eine ionenselektive Kunststoff-Membran (31,33) aufweist, die den Sensor-Elektrolyttank (40,42) abschließt und die jeweils in einer Öffnung der Außenwand des Kunststoff-Gehäuses (20) angeordnet ist.

2. Abwasseranalyse-Sensorkartusche (10) nach Anspruch 1, **dadurch gekennzeichnet, dass** die Kunststoff-Membran (31,33) stoffschlüssig mit dem Kunststoff-Gehäuse (20) verbunden ist.

3. Abwasseranalyse-Sensorkartusche (10) nach Anspruch 1 oder 2, **dadurch gekennzeichnet, dass** in den Öffnungen des Sensor-Elektrolyttanks (40,42) jeweils eine fluiddurchlässige Tragstruktur (35) angeordnet ist, wobei die Tragstruktur (35) jeweils die Membran (31,33) des betreffenden Sensorelementes (30,32) trägt.

4. Abwasscranalyse-Sensorkartusche (10) nach einem der Ansprüche 1-3, **dadurch gekennzeichnet, dass** die Gehäuseseitenwand (26) jeweils teilweise die Wand der Elektrolyttanks (40,42,44) bildet,

5. Abwasseranalyse-Sensorkartusche (10) nach einem der Ansprüche 1-4, **dadurch gekennzeichnet, dass** die Gehäuseseitenwand (26) becherförmig ist, wobei die Sensorelemente (30,32) an der Becher-Bodenwand (24) angeordnet sind.

6. Abwasseranalyse-Sensorkartusche (10) nach einem der Ansprüche 1 bis 5, **dadurch gekennzeichnet, dass** das Gehäuse (20) eine elektro-mechanische Kupplungsanordnung (70) aufweist, mit der die Sensorkartusche (10) an eine entsprechende elektr-o-mechanische Kupplungsanordnung (72) einer landseitig befestigten Haltevorrichtung (12) kuppelbar ist.

7. Abwasseranalyse-Sensorkartuschc (10) nach Anspruch 6, **dadurch gekennzeichnet, dass** die gehäuseseitige Kupplungsanordnung (70) ein Dichtelement (78,86) aufweist, so dass beide Kupplungsanordnungen (70,72) flüssigkeitsdicht aneinander kuppelbar sind.

8. Abwasseranalyse-Sensorkartusche (10) nach einem der Ansprüche 1 bis 7, **dadurch gekennzeichnet, dass** das Gehäuse (20) einen Temperatursensor (36) trägt.

9. Abwasseranalyse-Sensorkartusche (10) nach einem der Ansprüche 1 bis 8, **dadurch gekennzeichnet, dass** das Gehäuse (20) ein Stromschlüssel-Element (34) für das Referenzelement (56) trägt.

## Claims

1. A single-use waste water analysis sensor cartridge (10) comprising
an integral housing (20) of plastic material comprising at least two electrochemical sensor elements (30, 32) and at least one reference element (56), and
two sensor element electrolyte tanks (40, 42) and a reference element electrolyte tank (44), wherein
all electrolyte tanks (40, 42, 44) are formed by the integral housing (20), and
each electro-chemical sensor element (30, 32) comprises an ion-selective plastic membrane (31, 33) which closes the sensor electrolyte tank (40, 42) and is arranged in an opening of the outer wall of the plastic housing (20), respectively.

2. The waste water sensor cartridge (10) of claim 1, **characterized in that** the plastic membrane (31, 33) is integrally joined with the plastic housing (20).

3. The waste water sensor cartridge (10) of claim 1 or 2, **characterized in that** a fluid-permeable supporting structure (35) is arranged respectively in the openings of the sensor electrolyte tank (40, 42), the supporting structure (35) respectively supporting the membrane (31, 33) of the respective sensor element (30, 32).

4. The waste water sensor cartridge (10) of one of claims 1-3, **characterized in that** the side wall (26) of the housing forms a part of the wall of the electrolyte tanks (40, 42, 44), respectively.

5. The waste water sensor cartridge (10) of one of claims 1-4, **characterized in that** the side wall (26) of the housing is cup-shaped, wherein the sensor elements (30, 32) are arranged at the bottom wall (24) of the cup.

6. The waste water sensor cartridge (10) of one of claims 1-5, **characterized in that** the housing (20) comprises an electromechanical coupling arrangement (70) by means of which the sensor cartridge (10) can be coupled to a corresponding electromechanical coupling arrangement (72) of a retaining device (12) fastened on the land side.

7. The waste water sensor cartridge (10) of claim 6, **characterized in that** the housing-side coupling arrangement (70) comprises a sealing element (78, 86) so that both coupling arrangements (70, 72) can be coupled in a liquid-tight manner.

8. The waste water sensor cartridge (10) of one of claims 1-7, **characterized**
**in that** the housing (20) carries a temperature sensor (36).

9. The waste water sensor cartridge (10) of one of claims 1-8, **characterized in that** the housing (20) comprises a salt bridge element (34) for the reference element (56).

## Revendications

1. Cartouche de capteur (10) à utilisation unique, destinée à l'analyse des eaux usées, comprenant
un boitier monobloc (20) en matière plastique comprenant au moins deux éléments capteurs électrochimique (30, 32) et au moins un élément de référence (56), et
deux réservoirs d'électrolyte (40, 42) dudit élément capteur et un réservoir d'électrolyte (44) dudit élément de référence,
tous les réservoirs d'électrolyte (40, 42, 44) étant formés par ledit boitier monobloc (20), et
chaque élément capteur électrochimique (30, 32) comprenant une membrane sélective à ions (31, 33) en matière plastique qui ferme ledit réservoirs d'électrolyte du capteur (40, 42) et qui est disposée, respectivement, dans une ouverture dans la paroi extérieure du boitier (20) en matière plastique.

2. Cartouche de capteur (10) destinée à l'analyse des eaux usées selon la revendication 1, **caractérisée en ce que** la membrane (31, 33) en matière plastique est connectée de manière intégrale au boitier (20) en matière plastique.

3. Cartouche de capteur (10) destinée à l'analyse des eaux usées selon les revendications 1 ou 2, **caractérisée en ce qu'**une structure de support (35) perméable à liquide est disposée respectivement dans les ouvertures dudit réservoir d'électrolyte (40, 42) du capteur, ladite structure de support (35) respectivement portant la membrane (31, 33) dudit élément capteur (30, 32) respectif.

4. Cartouche de capteur (10) destinée à l'analyse des eaux usées selon l'une quelconque des revendications 1-3, **caractérisée en ce que** la paroi latérale (26) du boitier forme des parties respectives des réservoirs d'électrolyte (40, 42, 44).

5. Cartouche de capteur (10) destinée à l'analyse des eaux usées selon l'une quelconque des revendications 1-4, **caractérisée en ce que** la paroi latérale (26) est en forme de coupe, lesdits éléments capteur (30, 32) étant disposés sur la paroi de fond du coupe (24).

6. Cartouche de capteur (10) destinée à l'analyse des eaux usées selon l'une quelconque des revendications 1-5, **caractérisée en ce que** ledit boitier comprend un dispositif d'accouplement (70) électromécanique permettant l'accouplement de ladite cartouche de capteur (10) à un dispositif d'accouplement (72) électromécanique correspondant d'un dispositif de retenue (12) fixé côté terre.

7. Cartouche de capteur (10) destinée à l'analyse des eaux usées selon la revendication 6, **caractérisée en ce que** le dispositif d'accouplement (70), situé côté boitier, comprend un élément de joint (78, 86), de sorte que les deux dispositifs d'accouplement (70, 72) sont aptes à être couplés de manière étanche à liquides.

8. Cartouche de capteur (10) destinée à l'analyse des eaux usées selon l'une quelconque des revendications 1-7, **caractérisée en ce que** ledit boitier (20) porte un capteur de température (36).

9. Cartouche de capteur (10) destinée à l'analyse des eaux usées selon l'une quelconque des revendications 1-8, **caractérisée en ce que** ledit boitier (20) pore un élément pont salin (34) pour ledit élément de référence (56).
